# EUROPÄISCHE PATENTSCHRIFT

(11) **EP 0 825 977 B1**
(45) Veröffentlichungstag und Bekanntmachung des Hinweises auf die Patenterteilung: **16.02.2000**
(21) Anmeldenummer: 96919686.4
(22) Anmeldetag: 07.05.1996
(51) Int. Cl.: C07C 231/06, C07D 213/803, C07D 213/82, C07C 67/22

(54) **VERFAHREN ZUR HERSTELLUNG VON CARBONSÄUREDERIVATEN**
CARBOXYLIC ACID DERIVATIVE PREPARATION PROCESS
PROCEDE DE PREPARATION DE DERIVES D'ACIDE CARBOXYLIQUE

(30) Priorität: 19.05.1995 DE 19518474
(43) Veröffentlichungstag der Anmeldung: 04.03.1998
(73) Patentinhaber: BASF AKTIENGESELLSCHAFT, 67056 Ludwigshafen (DE)
(72) Erfinder: FUCHS, Eberhard, D-67227 Frankenthal (DE)
(86) Internationale Anmeldenummer: EP9601890
(87) Internationale Veröffentlichungsnummer: WO9636592

(56) Entgegenhaltungen:
- EP-A- 0 561 614
- WO-A-96/16039
- DE-A- 2 539 435
- US-A- 4 987 256
- US-A- 5 103 055
- CHEMICAL ABSTRACTS, vol. 78, no. 5, 5.Februar 1973 Columbus, Ohio, US; abstract no. 29639, KAIEDA, OSAMU ET AL: "Nicotinamide" XP002017426 & JP 47 031 984 A (JAPAN CATALYTIC CHEMICAL INDUSTRY CO., LTD.)

## Beschreibung

Die Erfindung betrifft ein Verfahren zur Herstellung von Carbonsäurederivaten aus Carbonsäurenitrilen und Alkoholen bei erhöhten Temperaturen an Titandioxid-Katalysatoren.

Aus der DE-A-27 14 767 ist ein Verfahren zur Herstellung von Carbonsäurederivaten durch Gasphasenreaktion von Nitrilen mit Alkoholen und Wasser an festen, metallhaltigen Katalysatoren wie z.B. Kupfer, Zink, Chrom, Wismut, Mangan, Eisen, Nickel, Cadmium auf Trägern wie Al₂O₃, SiO₂, ZrO₂ oder TiO₂ bekannt. Als Nebenreaktion findet man Dehydratisierung und Veretherung.

Beispielsweise aus der EP-A-412 310 ist ein Verfahren zur Herstellung von Carbonsäureamiden durch Hydratisierung von Nitrilen mit Wasser an Mangan-haltigen Katalysatoren mit Wasser bekannt, die sich anschließend mit Alkoholen durch Säurekatalyse, in Gegenwart von Metallsalzen z.B. nach US-A-4 613 684 oder in der Gasphase an festen Katalysatoren z.B. nach EP-A-561 614 oder mit Ameisensäureester unter Formamidbildung z.B. nach EP-A-392 361 an Metalloxiden zu den Carbonsäurederivaten umsetzen lassen.

Nachteilig auf die technische Anwendung wirkt sich die Zweistufigkeit und die Nebenproduktbildung wie Salzanfall oder Formamidbildung aus.

Aus der DE-A-43 39 648 ist ein Verfahren zur Herstellung von Caprolactam (ein cyclisches Amid) durch Umsetzung von Aminocapronsäurenitril mit Wasser in einer ethanolischen Lösung an einem Titanoxid-Katalysator bekannt.

Aus der US-A-5 103 055 ist ein Verfahren zur Herstellung von substituierten Amiden durch Umsetzung von Carbonsäurentrilen mit Alkoholen in Anwesenheit von Wasser bekannt.

Der vorliegenden Erfindung lag daher die Aufgabe zugrunde, den zuvor genannten Nachteilen abzuhelfen.

Demgemäß wurde ein neues und verbessertes Verfahren zur Herstellung von Carbonsäurederivaten der allgemeinen Formel I 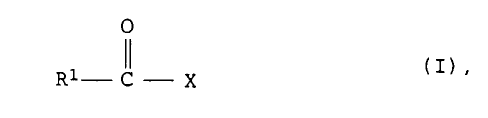 in der
- X: OR² oder NH₂,
- R¹: C₁- bis C₂₀-Alkyl, C₁- bis C₂₀-Hydroxyalkyl, C₃- bis C₁₂-Cycloalkyl, C₄- bis C₁₂-Alkyl-cycloalkyl, C₄- bis C₁₂-Cycloalkyl-alkyl, C₅- bis C₂₀-Alkyl-cycloalkyl-alkyl, Aryl, C₇- bis C₂₀-Aralkyl, C₇- bis C₂₀-Alkylaryl, einen heteroaliphatischen oder heteroaromatischen Ring mit 5- bis 8-Kohlenstoffatomen und
- R²: C₁- bis C₂₀-Alkyl
bedeuten, aus Carbonsäurenitrilen der allgemeinen Formel II

R¹― C ≡ N (II),

in der R¹ die oben genannten Bedeutungen hat, und Alkoholen der allgemeinen Formel III

R²―OH (III),

in der R² die oben genannten Bedeutungen hat, bei Temperaturen von 50 bis 300°C und Drücken von 0,1 bis 350 bar in Gegenwart eines Heterogenkatalysators in der Flüssigphase gefunden, welches dadurch gekennzeichnet ist, daß man als Katalysator Titandioxid einsetzt.

Das erfindungsgemäße Verfahren läßt sich wie folgt durchführen:

Das Carbonsäurenitril II kann mit einem Alkohol III, bevorzugt gelöst in dem Alkohol III bei Temperaturen von 50 bis 300°C, bevorzugt 100 bis 290°C, besonders bevorzugt 140 bis 270°C und Drücken von 0,1 bis 350 bar, bevorzugt 1 bis 200 bar, besonders bevorzugt 30 bis 140 bar in der Flüssigphase mit einen Titanoxid-Katalysator in der Regel in druckstabilen Apparaten wie Autoklaven und Rohrreaktoren, bevorzugt Rohrreaktoren in Kontakt gebracht werden. Man erhält in der Regel eine alkoholische Lösung des Carbonsäureesters und des Carbonsäureamids I aus der sich der Carbonsäureester und das Carbonsäureamid I nach üblichen Methoden, beispielsweise durch Destillation, Extraktion bzw. Kristallisation erhalten lassen.

Das Carbonsäureamid kann zusammen mit frischem Carbonsäurenitril II in den Reaktor zurückgefahren werden können.

Die Umsetzung fester Carbonsäurenitrile II bzw. fester Alkohole III läßt sich in inerte Lösungsmittel beipielsweise in Ethern, bevorzugt Ethern mit 2- bis 20-Kohlenstoffatomen, besonders bevorzugt Ethern mit 4- bis 12-Kohlenstoffatomen wie Diethylether, Methyl-tert.-butylether oder Tetrahydrofuran, Kohlenwasserstoffen, bevorzugt Kohlenwasserstoffen mit 5- bis 30-Kohlenstoffatomen, besonders bevorzugt Kohlenwasserstoffen mit 5- bis 12-Kohlenstoffatomen wie Toluol und Xylol oder vorteilhaft im entsprechenden Carbonsäureester I durchführen.

Als Heterogenkatalysatoren setzt man erfindungsgemäß Titandioxid, z.B. amorph, als Anatas oder Rutil oder deren Mischungen ein.

Die vorstehend genannten Verbindungen können mit Verbindungen der Gruppen IA, IIA, IIIA, IVA, VA, VIA und VIIA des Periodensystems der Elemente dotiert sein bzw. diese enthalten.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Die Katalysatoren können je nach der Zusammensetzung des Katalysators als Vollkontakt oder Trägerkatalysator verwendet werden. So kann Titandioxid als Titandioxid-Strang oder als auf einen Träger in dünner Schicht aufgebrachtes Titandioxid eingesetzt werden, Zum Aufbringen von TiO₂ auf einen Träger wie Siliciumdioxid, Aluminiumoxid oder Zirkondioxid sind alle in der Literatur beschriebenen Methoden verwendbar. So kann eine dünne TiO₂-Schicht durch Hydrolyse von Ti-Organylen wie Ti-Isopropylat oder Ti-Butylat, oder durch Hydrolyse von TiCl₄ oder anderen anorganischen Ti-haltigen Verbindungen aufgebracht werden. Auch Titanoxid-haltige Sole sind verwendbar.

Die Umsetzung kann beispielsweise in Suspensionsfahrweise, bevorzugt in Festbettfahrweise durchgeführt werden. Die Festbettfahrweise wird bevorzugt, da die Umsetzung damit auf einfache Weise kontinuierlich betrieben werden kann, die erzielten Ausbeuten und Selektivitäten im Festbett in der Regel sehr hoch sind und somit kurze Verweilzeiten mit sehr hohen Durchsätzen zur Folge haben. Da die verwendeten Heterogenkatalysatoren nach bisherigen Beobachtungen eine hohe Lebensdauer aufweisen, ergibt sich ein extrem geringer Katalysator-Verbrauch.

Die Substituenten X, R¹ und R² in den Verbindungen I, II und III haben folgende Bedeutungen:
X
   - OR²
   - NH₂
R¹, R²
   - C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl, insbesondere C₁- bis C₄-Alkyl wie Methyl, Ethyl, n-Propyl, iso-Propyl, n-Butyl, iso-Butyl, sec.-Butyl und tert.-Butyl.
   - Außerdem R¹
   - C₁- bis C₂₀-Hydroxyalkyl, bevorzugt C₁- bis C₈-Hydroxyalkyl, besonders bevorzugt C₁- bis C₄-Hydroxyalkyl wie Hydroxymethyl, 1-Hydroxyethyl, 2-Hydroxyethyl und 1-Methyl-1-hydroxyethyl,
   - C₃- bis C₁₂-Cycloalkyl, bevorzugt C₅- bis C₈-Cycloalkyl wie Cyclopentyl, Cyclohexyl, Cycloheptyl und Cyclooctyl, besonders bevorzugt Cyclopentyl und Cyclohexyl,
   - C₄- bis C₁₂-Alkyl-cycloalkyl, bevorzugt C₅- bis C₁₀-Alkyl-cycloalkyl, besonders bevorzugt C₅- bis C₈-Alkyl-cycloalkyl,
   - C₄- bis C₁₂-Cycloalkyl-alkyl, bevorzugt C₅- bis C₁₀-Cycloalkyl-alkyl, besonders bevorzugt C₅- bis C₈-Cycloalkyl-alkyl,
   - C₅- bis C₂₀-Alkyl-cycloalkyl-alkyl, bevorzugt C₆- bis C₁₆-Alkyl-cycloalkyl-alkyl, besonders bevorzugt C₇- bis C₁₂-Alkylcycloalkyl-alkyl,
   - Aryl wie Phenyl, 1-Naphthyl und 2-Naphthyl, bevorzugt Phenyl,
   - C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
   - C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl und 2-Phenylethyl,
   - einen heteroaliphatischen Ring mit 5- bis 8-Kohlenstoffatomen wie 2-Morpholinyl, 3-Morpholinyl, 2-Piperidinyl, 3-Piperidinyl und 4-Piperidinyl,
   - einen heteroaromatischen Ring mit 5- bis 8-Kohlenstoffatomen wie 2-Pyridyl, 3-Pyridyl, 4-Pyridyl, 2-Pyrazolyl, 2-Imidazolyl, 4(5)-Imidazolyl, bevorzugt 3-Pyridyl.

Die Carbonsäureester und die Carbonsäureamide I eignen sich als Zwischenprodukte, Kunststoffvorprodukte sowie im Pflanzenschutz und der Pharmazie.

### Beispiele

### Beispiel 1

Eine 20 Gew.-%ige ethanolische Lösung von Nicotinsäurenitril wurde unter Zusatz von 1 mol Wasser (3,5 Gew.-% bezogen auf die Lösung) bei 220°C und 80 bar durch ein mit Titanoxid (1,5 mm Stränge) gefülltes Rohr gefahren. Die Verweilzeit wurde über die Strömungsgeschwindigkeit variabel eingestellt.

Die Zusammensetzung des Reaktionsausstrages ist in Tabelle 1 zusammengestellt.

**Tabelle 1**

| Verweilzeit | Nicotinsäurenitril | Nicotinsäureethylester | Nicotinsäureamid | Sonstige |
|---|---|---|---|---|
| [min] | [%] | [%] | [%] | [%] |
| 15 | 5,1 | 5,4 | 9,4 | 0,1 |
| 30 | 1,9 | 8,6 | 9,1 | 0,4 |
| 60 | 0,6 | 11,3 | 7,4 | 0,7 |

### Vergleichsbeispiel 1

Analog Beispiel 1 wurde eine 20 Gew.-%ige ethanolische Lösung von Nicotinsäurenitril in Gegenwart von 1 mol Wassser bei 220°C und 80 bar durch ein leeres Rohr gepumpt. Es fand keine Reaktion statt.

## Patentansprüche

1. Verfahren zur Herstellung von Carbonsäurederivaten der allgemeinen Formel I 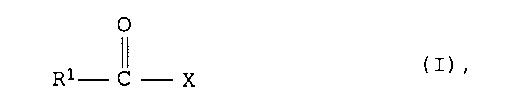 in der
X OR² oder NH₂,
R¹ C₁- bis C₂₀-Alkyl, C₁- bis C₂₀-Hydroxyalkyl, C₃- bis C₁₂-Cycloalkyl, C₄- bis C₁₂-Alkyl-cycloalkyl, C₄- bis C₁₂-Cycloalkyl-alkyl, C₅- bis C₂₀-Alkyl-cycloalkyl-alkyl, Aryl, C₇- bis C₂₀-Aralkyl, C₇- bis C₂₀-Alkylaryl, einen heteroaliphatischen oder heteroaromatischen Ring mit 5-bis 8-Kohlenstoffatomen und
R² C₁- bis C₂₀-Alkyl
bedeuten, aus Carbonsäurenitrilen der allgemeinen Formel II
R¹― C ≡ N (II),
in der R¹ die oben genannten Bedeutungen hat, und Alkoholen der allgemeinen Formel III
R²―OH (III),
in der R² die oben genannten Bedeutungen hat, bei Temperaturen von 50 bis 300°C und Drücken von 0,1 bis 350 bar in Gegenwart eines Heterogenkatalysators in der Flüssigphase, dadurch gekennzeichnet, daß man als Heterogenkatalysator Titandioxid einsetzt.

2. Verfahren zur Herstellung von Carbonsäurederivaten nach Anspruch 1, dadurch gekennzeichnet, daß man als Titandioxid Anatas, Rutil oder deren Gemische einsetzt.

3. Verfahren zur Herstellung von Carbonsäurederivaten nach den Ansprüchen 1 bis 2, dadurch gekennzeichnet, daß man die Umsetzung bei Temperaturen von 100 bis 290°C durchführt.

4. Verfahren zur Herstellung von Carbonsäurederivaten nach den Ansprüchen 1 bis 3, dadurch gekennzeichnet, daß man die Umsetzung bei Temperaturen von 140 bis 270°C durchführt.

5. Verfahren zur Herstellung von Carbonsäurederivaten nach den Ansprüchen 1 bis 4, dadurch gekennzeichnet, daß man die Umsetzung bei Drücken von 1 bis 200 bar durchführt.

6. Verfahren zur Herstellung von Carbonsäurederivaten nach den Ansprüchen 1 bis 5, dadurch gekennzeichnet, daß man die Umsetzung bei Drücken von 30 bis 140 bar durchführt.

7. Verfahren zur Herstellung von Carbonsäurederivaten nach Anspruch 1, dadurch gekennzeichnet, daß R¹ für 3-Pyridyl, 1-Methyl-1-hydroxyethyl, 1-Hydroxyethyl oder 2-Hydroxyethyl steht.

## Claims

1. A process for preparing carboxylic acid derivatives of the general formula I 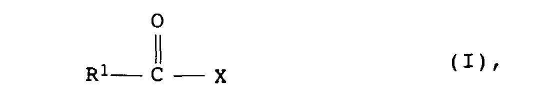 where
X is OR² or NH₂,
R¹ is C₁-C₂₀-alkyl, C₁-C₂₀-hydroxyalkyl, C₃-C₁₂-cycloalkyl, C₄-C₁₂-alkylcycloalkyl, C₄-C₁₂-cycloalkylalkyl, C₅-C₂₀-alkylcycloalkylalkyl, aryl, C₇-C₂₀-aralkyl, C₇-C₂₀-alkylaryl, a heteroaliphatic or heteroaromatic ring with 5 to 8 carbon atoms and
R² is C₁-C₂₀-alkyl,
from carbonitriles of the general formula II
R¹―C ≡ N (II),
where R¹ has the abovementioned meanings, and alcohols of the general formula III
R²―OH (III),
where R² has the abovementioned meanings, at from 50 to 300°C under from 0.1 to 350 bar in the presence of a heterogeneous catalyst in liquid phase, wherein titanium dioxide is used as heterogeneous catalyst.

2. A process for preparing carboxylic acid derivatives as claimed in claim 1, wherein anatase, rutile or mixtures thereof are used as titanium dioxide.

3. A process for preparing carboxylic acid derivatives as claimed in either of claims 1 and 2, wherein the reaction is carried out at from 100 to 290°C.

4. A process for preparing carboxylic acid derivatives as claimed in any of claims 1 to 3, wherein the reaction is carried out at from 140 to 270°C.

5. A process for preparing carboxylic acid derivatives as claimed in any of claims 1 to 4, wherein the reaction is carried out under from 1 to 200 bar.

6. A process for preparing carboxylic acid derivatives as claimed in any of claims 1 to 5, wherein the reaction is carried out under from 30 to 140 bar.

7. A process for preparing carboxylic acid derivatives as claimed in claim 1, wherein R¹ is 3-pyridyl, 1-methyl-1-hydroxyethyl, 1-hydroxyethyl or 2-hydroxyethyl.

## Revendications

1. Procédé de préparation de dérivés d'acide carboxylique de la formule générale 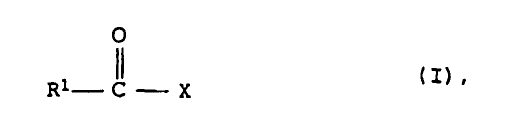 dans laquelle
X représente OR² ou NH₂,
R¹ représente un radical alkyle en C₁ à C₂₀, hydroxyalkyle en C₁ à C₂₀, cycloalkyle en C₃ à C₁₂, alkylcycloalkyle en C₄ à C₁₂, cycloalkylalkyle en C₄ à C₁₂, alkylcycloalkylalkyle en C₅ à C₂₀, aryle, aralkyle en C₇ à C₂₀, alkylaryle en C₇ à C₂₀, un noyau hétéroaliphatique ou hétéroaromatique comportant de 5 à 8 atomes de carbone et
R² représente un radical alkyle en C₁ à C₂₀,
à partir de nitriles carboxyliques de la formule générale II
R¹― C ≡N (II),
dans laquelle R¹ a les significations ci-dessus, et d'alcools de la formule générale III
R²―OH (III),
dans laquelle R² a les significations ci-dessus, à des températures de 50 à 300°C et des pressions de 0,1 à 350 bar, en présence d'un catalyseur hétérogène et en phase liquide, caractérisé en ce que d'on utilise du dioxyde de titane en tant que catalyseur hétérogène.

2. Procédé de préparation de dérivés d'acide carboxylique selon la revendication 1, caractérisé en ce que l'on utilise comme dioxyde de titane de l'anatase, du rutile ou leurs mélanges.

3. Procédé de préparation de dérivés d'acide carboxylique selon les revendications 1 à 2, caractérisé en ce que l'on entreprend la réaction à des températures de 100 à 290°C.

4. Procédé de préparation de dérivés d'acide carboxylique selon les revendications 1 à 3, caractérisé en ce que l'on entreprend la réaction à des températures de 140 à 270°C.

5. Procédé de préparation de dérivés d'acide carboxylique selon les revendications 1 à 4, caractérisé en ce que l'on entreprend la réaction sous des pressions de 1 à 200 bar.

6. Procédé de préparation de dérivés d'acide carboxylique selon les revendications 1 à 5, caractérisé en ce que l'on entreprend la réaction sous des pressions de 30 à 140 bar.

7. Procédé de préparation de dérivés d'acide carboxylique selon la revendication 1, caractérisé en ce que R¹ représente un radical 3-pyridyle, 1-méthyl-1-hydroxyéthyle, 1-hydroxyéthyle ou 2-hydroxyéthyle.
